# EUROPEAN PATENT APPLICATION

(11) **EP 4 389 002 A1**
(43) Date of publication of application: **26.06.2024**
(21) Application number: 22383249.4
(22) Date of filing: 21.12.2022
(51) Int. Cl.: A61B 5/16, A61B 5/22, G09B 15/06

(54) **HAND DEVICE FOR EVALUATING AND RECOVERING AFFECTED COGNITIVE AND MOTOR FUNCTIONS**

(71) Applicant: Fundacion Universidad Francisco de Vitoria (UFV), 28223 Pozuelo de Alarcón - Madrid (ES); Universidad Antonio de Nebrija, 28240 Hoyo de Manzanares Madrid (ES)
(72) Inventor: ROMERO, Juan Pablo, 28223 POZUELO DE ALARCÓN (Madrid) (ES); ÁLVAREZ FERNANDEZ, Roberto, 28240 HOYO DE MANZANARES (Madrid ) (ES); VALENZUELA LÓPEZ, Laura, 28223 POZUELO DE ALARCÓN (Madrid) (ES); MARTINEZ, Rodrigo, 28240 HOYO DE MANZANARES (Madrid ) (ES); DELGADO OLEAS, Gabriel Alfonso, 28223 POZUELO DE ALARCÓN (Madrid) (ES)
(74) Representative: Herrero & Asociados, S.L.

(57) **Abstract**

Hand device for evaluating and recovering affected cognitive and motor functions, the device having the shape of a cylinder with an inner hollow axis and being made up of four independent ring-shaped modules (1a-1d), the rings being capable of rotating independently about the main axis of the device, each ring being provided with a LED (4a-4d), a push button (2a-2d) and a corresponding pressure sensor (3a-3d), an analog to digital converter (5), and a sound emitting device for emitting a programmable multi tone signal, and a controller and microprocessor for receiving the signals generated by the pressure sensors and send signals to the emitters via cables, Bluetooth or Wi-Fi.

## Description

### BACKGROUND OF THE INVENTION

### Field of the Invention

The present invention relates to the field of medical devices. More in particular, the invention refers to a portable device for recovering and evaluating motor function of the hand, as well as cognitive function.

### Description of the Related Art

Cerebrovascular accidents, like stroke, are one of the leading causes of disability in adults. Most survivors experience some degree of motor deficit, which makes them unable to perform certain tasks in their daily lives. In addition to physical and motor disability, another very common consequence of stroke is the deterioration of cognitive functions, which also has a negative impact on the quality of life of patients and affects their daily life activities. There is prior evidence of a pathophysiological and structural correlation of cognitive and motor deficits among stroke survivors. Motor performance was associated with memory, executive function, and global cognition. Post-stroke rehabilitation that considers both motor and cognitive aspects is essential to achieve an optimal recovery of the patient and ensure the highest possible quality of life after the disease.

Currently, not only the evaluation of deficits, but also conventional rehabilitation is frequently supported by the use of robots and other devices to enhance rehabilitation. There are different devices available in clinical practice that serve as support in motor rehabilitation of the upper limb, the development of this type of device is increasing. Several of these devices are robots developed to assist and perform exercises that help recover lost functions. Most of these devices are mainly focused on the recovery of the shoulder and elbow segments, such as Armeo, from Hocoma AG, Switzerland (https://knowledge.hocoma.com/research) and Diego from Tyromotion, Austria (https://tyromotion.com/en/scientific-evidence) However, in cases where the motor function of the hand is severely affected, both its evaluation and rehabilitation are highly complex, since they involve not only the movement of one finger, but also movements generated by the coordination of different. The robots Pablo (Tyromotion, Austria) and Amadeo (Tyromotion, Austria) are robots used to train and also evaluate the strength of the arm-hand and hand-finger segments, respectively. These devices are the most used by brain injury rehabilitation centers, in combination with other therapies. However, due to their large size, weight, and cost, they are not feasible for home use, their application being limited to hospital environment under the supervision of a specialized therapist.

All these devices can also be used in the rehabilitation of diverse diseases or conditions that impair hand mobility, sensitivity or cognitive fitness, for example: Parkinson's Disease, Multiple Sclerosis, Dystonia, Arthritis, Dementia etc. All these diseases can be rehabilitated using the same devices described for brain injury rehabilitation and would benefit from a physical-cognitive approach for rehabilitation.

### SUMMARY OF THE INVENTION

The invention solves the problems above by providing a system that helps recovering the affected cognitive and motor functions simultaneously and does so with a device that is portable, easy to use at home and adjustable to each patient. For this purpose, the invention provides a modular device that interacts with the fingers independently. The device has the shape of a cylinder and is made up of four independent ring-shaped modules that are joined and snapped together. Each ring module is provided with a pushbutton at its surface associated with at least a pressure sensor and LEDs or a sound emitting device that emits a programmable signal. The rings can be rotated independently about the main axis of the device and thus, the latter can be manipulated by people with very different hand sizes, resulting in an ergonomic design that allows adjustment of the position of each pushbutton to the position of each finger. Electrical connections between the push buttons, LEDs and sound emitter and a microprocessor are housed inside the device. A battery and an analog to digital converter, as well as the sound emitting device that emits a programmable multi tone signal are also housed inside the device (axis) and covered by the rings, in the hollow space formed by the rings. The controller and microprocessor could be provided as separated modules for receiving the signals generated by the buttons and send signals to the emitters (light, sound and or vibration) via Bluetooth or Wi-Fi.

Each ring can be manufactured in a variety of materials as metal, silicone, aluminum, or steel. Plastics like PLA and ABS are preferred, so that the rings can be manufactured by 3D printers. The width of each ring is set between 1 and 4 cm, therefore when the four ring rings are in place, the device has a total perimeter which is approximately the length of the hand avoiding overlapping the thumb and index. The rings have a diameter of 4-9 cm.

### BRIEF DESCRIPTION OF THE DRAWINGS

To complete the description for a better understanding of the invention, a set of drawings is provided. These drawings illustrate a preferred embodiment of the invention, which should not be interpreted as restricting the scope of the invention, but just as an example of how the invention can be carried out.
Figures 1a and 1b show one of the rings. The rings are the basic components of the invention.
Figure 2 shows the device with all four rings.

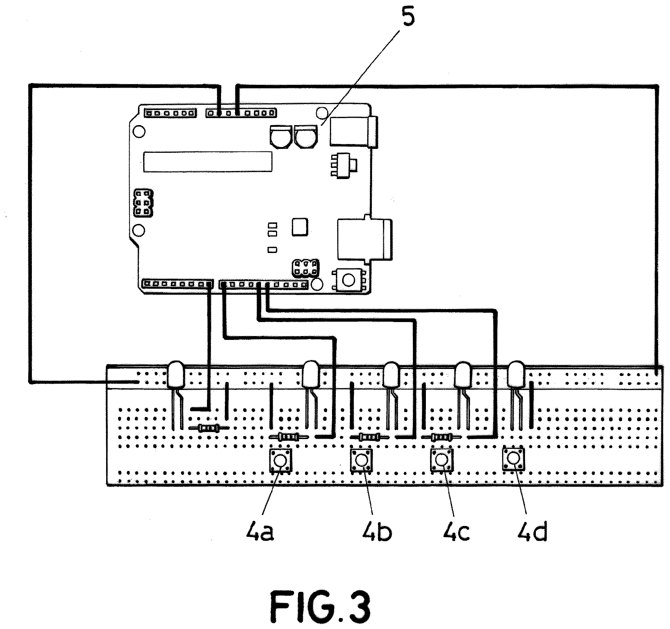

Figure 3 is a scheme of the sensors and emitters coupled to the controller as in the present invention.

### DESCRIPTION OF THE INVENTION

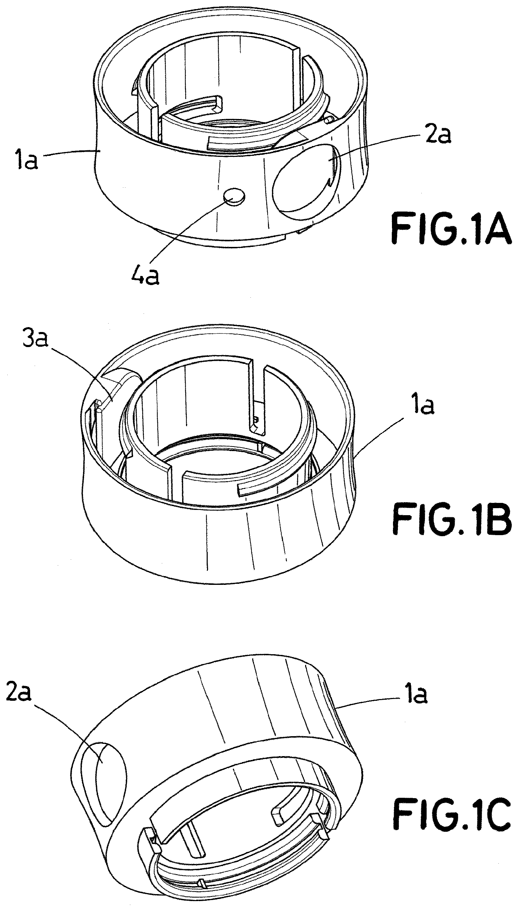

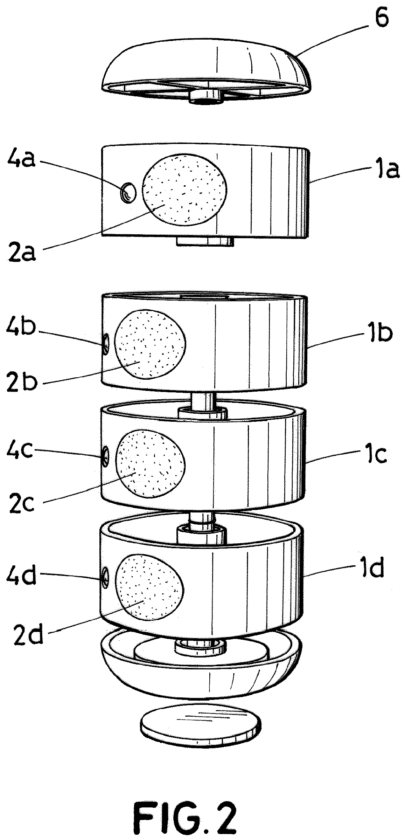

With reference to figures 1a, 1b and figure 2, the portable device of the invention is provided with four concentric rings (1a-1d) arranged around a common axis. The rings can rotate independently to a certain extent and are provided at their external surface with pushbuttons (2a-2d). In figures 1a and 1b, only the aperture intended to house the pushbuttons is shown. Underneath each push button, a pressure sensor (3a-3d) is placed and connected to an analog to digital converter (5) housed inside the device, in the inner space formed by the rings, usually at one end close to a closing cup (6).

Each pushbutton is associated with a LED emitter (4a-4d). Also housed inside, there is a sound emitter coupled to each and all the pushbuttons.

With reference to figure 1a, the inner aperture of the ring is provided with a pressure relief and two tabs separated 45°-90°. The pressure relief and tabs provide for a snug fitting while assuring that the rings can be rotated (to a certain extent, allowing a 45 to 90 degrees spin), so that the device can be adjusted to different finger lengths without interfering with the internal mechanism.

The interaction between the patient and the device is established by means of a sequence of lights and/or sounds associated with a corresponding pushbutton to be pressed in response to a previously programmed light or sound. The pushbuttons have built-in pressure sensors that determine the force with which the pushbutton is pressed and the stroke of the pushbutton.

The patient will press the push button in response to a predetermined stimulus set by a colour/sound string. The colour/sound is displayed/heard by a series of light and/or sound indicators (also referred as elements). The subject must press the button or not depending on the stimulus. The system facilitates the management of the information obtained through the interaction response with a pre-programmed protocol of lights/sounds. Signalling the error and the success of the user's responses.

Each LED indicator can light up within a range of colours whose programmed sequence of combinations is reactive to the subject's response.

The device includes an intelligent system based on a Raspberry, Arduino or similar. microprocessor that allows to display the different training protocols and store them to finally proceed to the evaluation.

Sound and visual signals will be the outputs of the system. The subject's response after the performance of each of the tests will be evaluated considering the following points:
- The device collects data from the interaction with the subject and it can display such data both, raw and processed, through a web platform or a mobile application that will allow viewing in a mobile phone, a tablet, or a computer screen. The platform or mobile application will also allow the input of data to the microprocessor for generating the signals sent to the LEDs and sound emitter.

- Data collected by the microprocessor from each patient can be stored locally and can also be sent to a cloud storage system via wi-fi or Bluetooth.
- The data obtained allow the cognitive and physical evaluation of the patients through their interaction with the device.

The microprocessor stores and sends different types of data from the subject's interaction continuously to a web platform or app housed outside the device:
- Intensity or strength: to measure the force/pressure/tension performed by each finger over the sensor
- Timing of interaction: The time elapsed between the application of the stimulus (light or sound) and the pressing of the finger. Time the sensor remains pressed
- Frequency and sequence of interaction: registers the sequence in which buttons are pressed.
In a further embodiment, the device can also be provided with a gyroscope and a vibration generator unit housed inside the axis.

The device has two main pre-programmed modalities that are training and evaluation. Within each one of them there are different pre-established programs to train or evaluate varied motor and cognitive functions The user can choose to execute one of these programs or to use a fully customizable and programmable operating mode. Examples of the programs using the LEDs as output are presented in the following, although similar exercises can be performed with sound instead of lights

### Training mode:

- Exercise 1: the user can press the force sensors of the four fingers individually or simultaneously. Depending on the force with which each button is pressed, the corresponding LED emits a colour, this light will remain on for as long as the user is pressing the button. The colour depends on the force, so three ranges of grip force are distinguished: if a button is pressed with a small force, the corresponding LED will emit red light; if a button is pressed with a high force, the corresponding LED will emit other colour (green, for example); if a button is pressed with an intermediate force, the LED will emit another colour (yellow, for example). The change in tonality can be progressive. On the other hand, if the four buttons are pressed simultaneously with the same force, the four LEDs will emit a blue light. The device records and stores every interaction. The exercise will finish one each button has been pressed at least four times.

- Exercise 2: after a double flash of white light emitted by the four LEDs simultaneously (which indicates the beginning of the exercise) the device will emit blue lights one by one following a certain sequence. Once a LED turns blue, it will not turn off until the user presses its corresponding button. If the user presses an incorrect button, the device keeps the blue light waiting for the correct press. When the user presses the correct button, the blue light turns off, quickly turning green, yellow, or red, depending on how much time has been taken to press since the blue light turned on. The LED will turn green if the user presses the button quickly, it will light red if the time is long and it will turn yellow if the time to press is an intermediate value between the previous ranges. Once the user releases the correct button, the next LED in the sequence will turn blue, following the same mechanism as just described. Once the last button in the sequence has been pressed, the device will once again emit a double white flash to indicate that the exercise has ended. The device records and stores every interaction. This exercise has three levels of difficulty:
   - Easy: it is a simple sequence, without repetitions of fingers, only consecutive fingers.
   - Medium: it is a sequence in which the order of some fingers is inverted.
   - Difficult: in this sequence there exists finger jumps and repetitions.
- Exercise 3: after a double flash of white light emitted by the four LEDs simultaneously, the device will emit blue light following a defined sequence. The first LED in the sequence will turn on for two seconds, then this LED will turn off and the next LED in the sequence will turn on. This sequence will continue until the exercise is completed. The device will emit a double flash of white light from all four LEDs simultaneously to indicate that the sequence has ended. After this, the user will have to press the buttons in the same order in which the sequence of LEDs turned on. If the pressed button is correct, its corresponding LED will emit blue light, if it is incorrect, the LED will emit red light. When the user has pressed a number of buttons equal to the number of elements in the sequence, the exercise is finished. The device records and stores every interaction. Difficulty in the exercise can be low or medium when using three or five elements in the sequence and could even be increased.

### Evaluation mode:

- Exercise 1: the user can press all four fingers individually or simultaneously. Depending on the force with which each button is pressed, the device emits a light with a certain colour through the LED of that same cylinder, this light will remain on for as long as the user is pressing the button. The colour depends on the force, so three ranges of grip force are distinguished: if a button is pressed with a small force, the corresponding LED will emit red light; if a button is pressed with a high force, the corresponding LED will emit green light; if a button is pressed with a force intermediate to the previous ranges, the LED will emit yellow light. On the other hand, if the four buttons are pressed simultaneously with the same range of force, the four LEDs will emit blue light. For each user press, the device records and stores the maximum force reached and the corresponding finger. The exercise will finish once each button has been pressed at least four times.
- Exercise 2: after a double flash of white light by the four LEDs simultaneously, the device will emit blue light thanks to the different LEDs following a sequence. Once a LED turns blue, it will not turn off until the user presses its corresponding push button. If the user presses an incorrect one, the device keeps the same LED in blue, waiting for the correct press, and it will register and store two data: this press will be counted as an error, also indicating which finger corresponds to the error, and on the other hand it will save the maximum force to which that button was pressed. When the user correctly presses the corresponding push button, the blue light turns off, and the device registers and stores two values: the time in milliseconds that the user has taken to press the push button since the LED turned blue, and the maximum force reached in that pulsation. Once the user releases the correct button, the next LED in the sequence will turn blue, following the same procedure as just described. Once the last button in the sequence has been pressed, the device will once again emit a double white flash to indicate that the exercise has ended. In addition, the device records and stores every interaction. This exercise can also have three levels of difficulty:
   - Easy: it is a simple sequence, without repetitions of fingers, only consecutive fingers.
   - Medium: it is a sequence in which the order of some fingers is inverted.
   - Difficult: in this sequence there are finger jumps and repetitions.
- Exercise 3: after a double flash of white light by the four LEDs simultaneously, the device will emit blue light thanks to the different LEDs following a defined sequence. The first LED in the sequence will turn on for two seconds, then this LED will turn off and the next LED in the sequence will turn on, and this procedure will continue until the exercise sequence is completed. The device will emit a double flash of white light from all four LEDs simultaneously to indicate that the sequence has ended. After this, the user will have to press the buttons in the same order in which the sequence of LEDs turned on. If the sequence is wrong, the device will log and store it as an error, indicating how many buttons have been missed. When the user has pressed a number of buttons equal to the number of elements in the light sequence, the exercise ends. In addition, the device records and stores every interaction. This exercise can have different levels of difficulty, depending on the elements of the sequence.

As it is used herein, the term "comprises" and derivations thereof (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.) to be within the general scope of the invention as defined in the claims.

## Claims

1. Hand device for evaluating and recovering affected cognitive and motor functions, the device having the shape of a cylinder with an inner hollow axis and being made up of four independent ring-shaped modules (1a-1d) that are joined and snapped together, the rings being capable of rotating independently about the main axis of the device, each ring being provided with a LED (4a-4d), a push button (2a-2d) and a corresponding pressure sensor (3a-3d), and a controller and microprocessor for receiving, storing and sending the signals generated by the pressure sensors and send signals to the LEDs via Bluetooth or Wi-Fi.

2. Hand device for evaluating and recovering affected cognitive and motor functions according to claim 1 further comprising a sound emitting device for emitting a multi tone signal in response to the microprocessor signals.

3. Hand device for evaluating and recovering affected cognitive and motor functions according to claims 1 or 2, further provided with a gyroscope and means for generating a vibration.
